# EUROPÄISCHE PATENTSCHRIFT

(11) **EP 1 648 894 B1**
(45) Veröffentlichungstag und Bekanntmachung des Hinweises auf die Patenterteilung: **12.09.2007**
(21) Anmeldenummer: 04741219.2
(22) Anmeldetag: 22.07.2004
(51) Int. Cl.: C07D 473/00, A61K 31/52, A61P 31/12

(54) **VERFAHREN ZUR HERSTELLUNG VON OH-GESCHÜTZTEN [4-(2,6-DIAMINO-9H-PURIN-9-YL)-1,3-DIOXOLAN-2-YL]METHANOL-DERIVATEN**
METHOD FOR THE PRODUCTION OF OH PROTECTED [4-(2.6-DIAMINO-9H-PURINE-9-YL)- 1.3-DIOXOLANE-2-YL]METHANOL DERIVATIVES
PROCEDE POUR PRODUIRE DES DERIVES DE [4-(2,6-DIAMINO-9H-PURINE-9-YL)-1,3-DIOXOLAN-2-YL]METHANOL A PROTECTION OH

(30) Priorität: 31.07.2003 DE 10335061
(43) Veröffentlichungstag der Anmeldung: 26.04.2006
(73) Patentinhaber: Wacker Chemie AG, 81737 München (DE)
(72) Erfinder: DÖRING, Wolfgang, 84561 Mehring (DE); PETERSEN, Hermann, 84489 Burghausen (DE)
(74) Vertreter: Fränkel, Robert
(86) Internationale Anmeldenummer: PCT/EP2004/008197
(87) Internationale Veröffentlichungsnummer: WO 2005/012302

(56) Entgegenhaltungen:
- EP-A- 1 258 486
- WO-A-01/58894
- EVANS C A ET AL: "DIVERGENT ASYMMETRIC SYNTHESES OF DIOXOLANE NUCLEOSIDE ANALOGUES" TETRAHEDRON: ASYMMETRY, ELSEVIER SCIENCE PUBLISHERS, AMSTERDAM, NL, Bd. 4, Nr. 11, 1993, Seiten 2319-2322, XP001145856 ISSN: 0957-4166
- KIM H O ET AL: "1,3-DIOXOLANYLPURINE NUCLEOSIDES (2R,4R) AND (2R,4S) WITH SELECTIVE ANTI-HIV-1 ACTIVITY IN HUMAN LYMPHOCYTES" JOURNAL OF MEDICINAL CHEMISTRY, AMERICAN CHEMICAL SOCIETY. WASHINGTON, US, Bd. 36, Nr. 1, 1993, Seiten 30-37, XP001146516 ISSN: 0022-2623
- KIM H O ET AL: "L-SS-(2S,4S)- AND L-A-(2S,4R)-DIOXOLANYL NUCLEOSIDES AS POTENTIAL ANTI-HIV AGENTS: ASYMMETRIC SYNTHESIS AND STRUCTURE-ACTIVITY RELATIONSHIP" JOURNAL OF MEDICINAL CHEMISTRY, AMERICAN CHEMICAL SOCIETY. WASHINGTON, US, Bd. 36, Nr. 5, 1993, Seiten 519-528, XP000887154 ISSN: 0022-2623

## Beschreibung

Die Erfindung betrifft ein Verfahren zur Herstellung von [4-(2,6-diamino-9*H*-purin-9-yl)-1,3-dioxolan-2-yl]methanol-Derivaten enthaltend eine Schutzgruppe R¹ an der Hydroxylgruppe (im folgenden kurz Hydroxyschutzgruppe bzw. "OH-geschützt(e)/(en)") der allgemeinen Formel (1), wobei die Reste R⁸, R⁹, R¹⁰ und R¹¹ unabhängig voneinander für Wasserstoff oder eine Aminoschutzgruppe stehen.

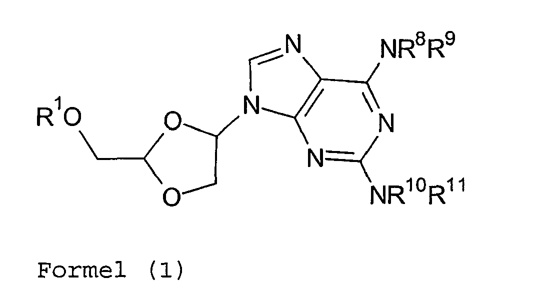

Nucleoside und Nucleosidanaloga stellen eine wichtige Klasse von antiviral wirksamen Substanzen dar. Beispiele für Nucleosidanaloga, die Wirksamkeit gegen HIV zeigen, sind 3'-Azido-3'-deoxythymidin (AZT) und 2',3'-Dideoxycytidin (ddC). Aufgrund von verschiedenen Effekten, vor allem aber wegen des Aufkommens von Resistenzen, wurden modernere Substanzen mit verändertem Wirkprofil entwickelt.

Als besonders vorteilhaft haben sich solche Nucleosidanaloga erwiesen, die einen 1,3-Oxathiolanring wie z.B. Lamivudin (3TC) und Coviracil (FTC) oder einen 1,3-Dioxolanring wie [(2*R*, 4*R*)-[4-(2,6-diamino-9*H*-purin-9-yl)-1,3-dioxolan-2-yl]methanol (im folgenden kurz "(-)-DAPD") enthalten.
Für die Synthese solcher Nucleosidanaloga stellt die Umsetzung der Nucleobase bzw. ihres synthetischen Vorläufers mit dem Zuckerbaustein in Gegenwart einer Lewis-Säure mittlerweile eine aus dem Stand der Technik bekannte Standardreaktion dar. Verwendet man dabei eine silylierte Nucleobase, ist die Reaktion als "Silyl-Hilbert-Johnson-Reaktion" bekannt [H. Vorbrüggen, G. Hoefle, Chem. Ber. 1981, 114, 1256-1268].

Als Lewis-Säuren verwendet man neben Metall- und Übergangsmetallhalogeniden und -alkoholaten wie z.B. SnCl₄, TiCl₄ oder TiCl₂(OiPr)₂ häufig auch Silylderivate von Perfluorsulfonsäuren wie z.B. Trifluormethansulfonsäuretrimethylsilylester oder Trialkylsilylhalogenide wie z.B. Iodtrimethylsilan.

Mechanistisch geht man im Falle von Ribosederivaten (d.h. 2'-substituierten Zuckerbausteinen) davon aus, dass sich aus dem Zuckerbaustein enthaltend eine Abgangsgruppe in 1'-Position, wie z.B. Acetat unter Einfluss der Lewis-Säure durch Nachbargruppeneffekt ein Kation ausbildet, das im zweiten Schritt mit der silylierten Nucleobase reagiert.Im Falle von Silylhalogeniden wie Iodtrimethylsilan wird in WO 01/58894 zunächst der Austausch der Abgangsgruppe durch Halogenid, wie z.B. Iodid, postuliert. Anschließend wird die gebildete Iodverbindung dann mit der silylierten Nucleobase umgesetzt.

Zur Synthese von Nucleosidanaloga, welche 2,6-Diaminopurin als Base enthalten, wird normalerweise zunächst 2,6-Dichlorpurin oder 2-Amino-6-chlorpurin als Basenvorläufer eingefügt und in einem späteren Schritt das / die Chloratom/e in Aminogruppen überführt. Dies kann direkt durch Umsetzung mit Ammoniak geschehen oder in zwei Schritten durch Umsetzung mit Azid zum Diazidoderivat und anschließender katalytischer Hydrierung zum Diaminoderivat. Die direkte Umsetzung mit Ammoniak gelingt nur schlecht unter Erzielung sehr niedriger Ausbeuten. Die Azid-Variante hat den Nachteil, dass zwei Reaktionsschritte benötigt werden. Von großem Nachteil in beiden Verfahren ist jedoch, dass der Einsatz des sehr teueren 2,6-Dichlorpurin bzw. 2-Amino-6-chlorpurin, die Reaktion wirtschaftlich uninteressant macht. Zusätzlich muss man aufgrund der höheren Molgewichte der Edukte in den beschriebenen Reaktionsvarianten statt 1 kg Diaminopurin ca. 1.25 kg Dichlorpurin bzw. 1.13 kg Aminochlorpurin (gleiche Ausbeuten vorausgesetzt) einsetzen.

In WO 97/21706 wird ein Verfahren zur Herstellung von β-Nucleosidanaloga mit 1,3-Dioxolanring beschrieben, wobei eine Purin- oder Pyrimidinbase bei Temperaturen unterhalb von -10°C mit einem 1,3-Dioxolanbaustein umgesetzt wird, der als Abgangsgruppe ein Halogenatom enthält. Der Dioxolanbaustein wird hierbei vorzugsweise aus dem entsprechenden Acetoxyderivat durch Umsetzung mit Iodtrimethylsilan oder Diiodsilan hergestellt.
Dabei wird besonders auf die hohe Stereoselektion bei der Durchführung bei tiefen Temperaturen hingewiesen.
Nachteilig bei diesem Verfahren ist, dass man auf Tieftemperaturreaktionen angewiesen ist, denn besonders hohe Stereoselektivitäten (β:α-Isomerenverhältnis) werden bei einer Reaktionstemperatur von -78°C beschrieben.
Verwendet man 2,6-Diaminopurin als Base, so erhält man nach diesem Verfahren nur schlechte Ausbeuten bzw. zahlreiche Nebenprodukte (vgl. Vergleichsbeispiel 5). Ferner sind aufgrund der geringen Reaktivität von Diaminopurin niedrige Reaktionstemperaturen, die jedoch nach der Lehre von WO 97/21706 für das Erzielen hoher Stereoselektivitäten notwendig sind, verfahrenstechnisch von großem Nachteil, da diese sehr lange Reaktionszeiten (>24 h) bedingen.

In WO 01/58894 wird die Herstellung von DAPD bzw. seines Enantiomeren beschrieben, in dem das aus WO 97/21706 bekannte Verfahren auf die Umsetzung von 4-Acetoxy-2-benzoyloxymethyl-1,3-dioxolan mit 2-Amino-6-Chlorpurin (Durchführung bei -15°C) angewandt wird. Das durch Säulenchromatographie gereinigte Produkt mit einem Isomerenverhältnis von β:α = 2.3:1 wird anschließend durch Umsetzung mit methanolischem Ammoniak und nachfolgender Säulenchromatographie in DAPD mit einem Isomerenverhältnis von β:α = 2:1 überführt. Nachteilig ist hier wiederum die Verwendung von teurem 2-Amino-6-Chlorpurin und der mehrfache Einsatz von Säulenchromatographie.

Der Erfindung lag die Aufgabe zugrunde, ein kostengünstiges und großtechnisch einfach umsetzbares Verfahren zur Herstellung von OH-geschützten [4-(2,6-diamino-9H-purin-9-yl)-1,3-dioxolan-2-yl]methanol-Derivaten in racemischer oder optisch reiner Form bereitzustellen, welches auf der direkten Umsetzung von 2,6-Diaminopurin bzw. eines ein- oder mehrfach silylierten 2,6-Diaminopurins oder deren Derivaten basiert.

Es wurde nun überraschend gefunden, dass die direkte Umsetzung von 2,6-Diaminopurin bzw. eines ein- oder mehrfach silylierten 2,6-Diaminopurins oder deren Derivate mit hohen chemischen Ausbeuten und ggf. hoher Stereoselektivität ohne aufwändige Reinigungsschritte gelingt, wenn bei der Glycosylierungsreaktion in der Reaktionsmischung mindestens ein Hilfsstoff in Form einer 1,3-Dicarbonylverbindung oder eines silylierten Derivates einer 1,3-Dicarbonylverbindung zugegen ist.

Gegenstand der Erfindung ist ein Verfahren zur Herstellung von Verbindungen der allgemeinen Formel (1) 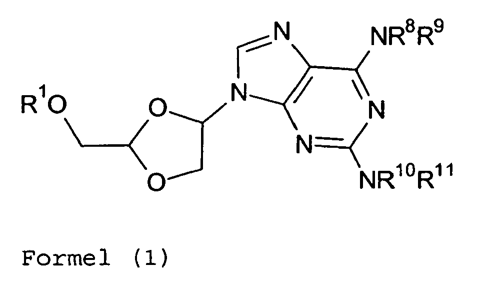 wobei
**R¹** für eine Hydroxyschutzgruppe und
**R⁸, R⁹, R¹⁰, R¹¹** unabhängig voneinander ausgewählt werden aus der Gruppe enthaltend Wasserstoff oder eine Aminoschutzgruppe
durch Umsetzung einer Verbindung der allgemeinen Formel (2) 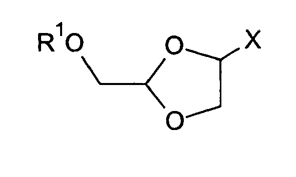 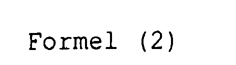 wobei
**X** für eine Abgangsgruppe steht
mit einem 2,6-Diaminopurinderivat der allgemeinen Formel (5) 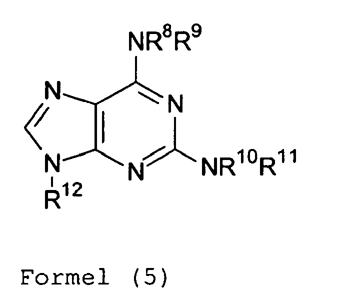 wobei
**R¹²** für einen Silylrest steht
in Gegenwart einer Lewis-Säure, dadurch gekennzeichnet, dass
zusätzlich eine 1,3-Dicarbonylverbindung oder ein silyliertes Derivat einer 1,3-Dicarbonylverbindung zugegen ist, wobei als 1,3-Dicarbonylverbindung ein β-Carbonylcarbonsäureester, ein 1,3-Diketon oder ein Malonsäurederivat mit 5 bis 20 C-Atomen der allgemeinen Formel (3) verwendet wird, 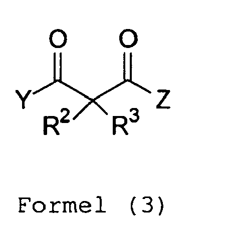 wobei
**Y** und **Z** unabhängig voneinander Wasserstoff, einen Alkylrest mit 1 bis 20 C-Atomen, einen Arylrest mit 6 bis 20 C-Atomen oder eine Alkyloxygruppe mit 1 bis 20 C-Atomen und
**R²** und **R³** unabhängig voneinander Wasserstoff, einen Acylrest einer aromatischen oder aliphatischen Carbonsäure mit 2 bis 20 C-Atomen, einen Alkylrest mit 1 bis 20 C-Atomen oder einen Arylrest mit 6 bis 20 C-Atomen
bedeuten können.

Durch das erfindungsgemäße Verfahren ist es möglich in einer großtechnische einfach realisierbaren Reaktion direkt kostengünstige 2,6-Diaminopurinderivate als Edukte einzusetzen ohne die Reaktion bei großtechnisch schwierig zu realisierenden Tieftemperaturbedingungen durchführen zu müssen oder die erhaltenen Rohprodukte durch anschließende aufwändige Aufarbeitungsverfahren von unerwünschten Nebenprodukten zu befreien.

Das erfindungsgemäße Verfahren ist dabei für die Herstellung racemischer Verbindungen der allgemeinen Formel (1) wie auch zur Herstellung von optisch reinen Produkten der allgemeinen Formel (1) mit den optischen Konfigurationen der allgemeinen Formeln (1a), (1b), (1c) und (1d) anwendbar.

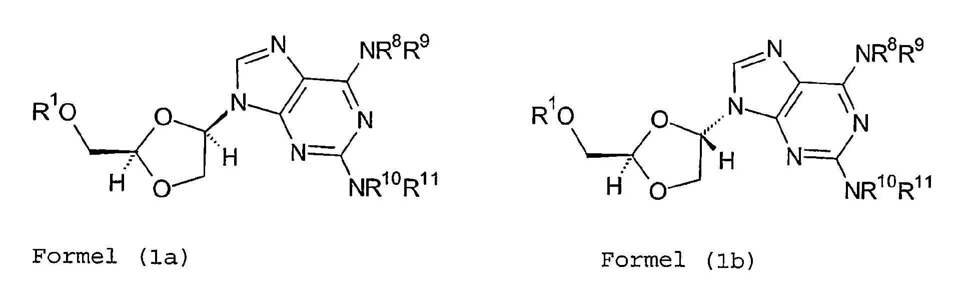

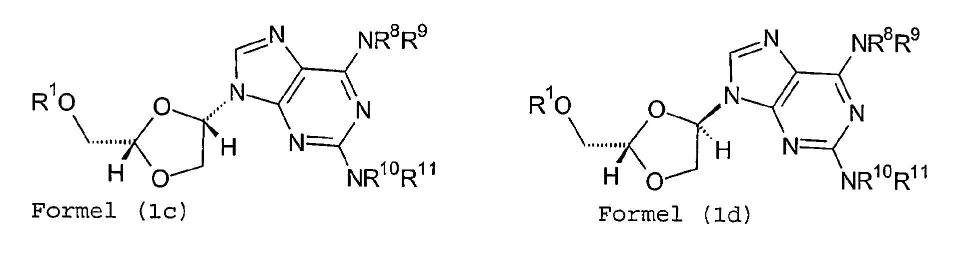

Durch Auswahl entsprechend optisch konfigurierter Edukte lässt sich das Verfahren mit hoher Stereoselektivität durchführen.

Insbesondere eignet sich das erfindungsgemäße Verfahren zur Herstellung von Produkten in der optischen Konfiguration der allgemeinen Formel (1a).

Die Hydroxyschutzgruppe R¹ kann dabei aus allen dem Fachmann geeigneten und bekannten OH-Schutzgruppen ausgewählt werden; eine Auswahl geeigneter OH-Schutzgruppen ist insbesondere in T.W. Greene, P.G.M. Wuts, "Protective Groups in Organic Synthesis", 2nd Edition, Wiley 1991, S. 10-117 beschrieben.

Vorzugsweise werden die Hydroxyschutzgruppen R¹ aus der Gruppe enthaltend Acylreste, Alkylreste, Alkoxyalkylreste, Arylalkylreste, Arylalkoxyalkylreste oder Silylreste ausgewählt.

Acylreste für R¹ leiten sich dabei bevorzugt aus einer aromatischen oder aliphatischen Carbonsäure mit 2 bis 20 C-Atomen ab, besonders bevorzugt aus der Gruppe enthaltend Benzoyl-, n-Butyryl-, Isobutyryl- (2-Methylpropionyl-), Pivaloyl-, Propionyl- und Acetyl-Reste.

Alkylreste für R¹ bestehen bevorzugt aus 1 bis 20 C-Atomen, besonders bevorzugt sind Reste aus der Gruppe enthaltend Methyl-, Ethyl- und Propyl-Reste.

Alkoxyalkylreste für R¹ bestehen bevorzugt aus 1 bis 20 C-Atomen, besonders bevorzugt sind Reste aus der Gruppe enthaltend Methoxymethyl-, 1-Ethoxyethyl- und 2-Methoxyethoxymethyl-Reste.

Arylalkylreste für R¹ bestehen bevorzugt aus 1 bis 20 C-Atomen, besonders bevorzugt sind Reste aus der Gruppe enthaltend Benzyl-, 4-Methoxybenzyl- und Triphenylmethyl-Reste.

Arylalkoxyalkylreste für R¹ bestehen dabei bevorzugt aus 1 bis 20 C-Atomen, besonders bevorzugt sind Reste aus der Gruppe enthaltend Benzyloxymethyl- und 4-Methoxybenzyloxymethyl-Reste.

Silylreste für R¹ können am Si-Atom generell aliphatische und/oder aromatische Substituenten mit jeweils 1 bis 20 C-Atomen, insbesondere mit jeweils 1 bis 10 C-Atomen enthalten. Bevorzugt sind Reste aus der Gruppe enthaltend Trimethylsilyl-Triethylsilyl-, Triisopropylsilyl-, *tert*-Butyldimethylsilyl- und *tert*-Butyldiphenylsilyl-Reste.

Die Abgangsgruppe X ist nach J. March, "Advanced Organic Chemistry", 3rd Edition, Wiley 1985, S. 179 und 310ff als Teil eines Substrat-Moleküls definiert, welches bei einer Reaktion abgespalten wird. Es wird dabei von einem "Nucleofug" gesprochen, wenn die Abgangsgruppe das Elektronenpaar der gespaltenen Bindung mit sich trägt.

Vorzugsweise werden Abgangsgruppen X aus der Gruppe enthaltend Halogen, Acyloxyrest, Alkylsulfonyloxyrest, Arylsulfonyloxyrest, Alkoxyrest oder Aryloxyrest ausgewählt.

Halogene für X sind bevorzugt Iod oder Brom, besonders bevorzugt ist Iod.

Acyloxyreste für X bestehen dabei bevorzugt aus 1 bis 20 C-Atomen, besonders bevorzugt sind Reste aus der Gruppe enthaltend Acetoxy-, Benzoyloxy-, Propionyloxy-, n-Butyryloxy- und Trifluoracetoxy-Reste. Ganz besonders bevorzugt ist der Acetoxy-Rest.

Alkylsulfonyloxyreste für X bestehen dabei bevorzugt aus 1 bis 20 C-Atomen, besonders bevorzugt sind Reste aus der Gruppe enthaltend Methansulfonyloxy-, Trifluormethansulfonyloxy- und Nonafluorbutylsulfonyloxy-Reste.

Arylsulfonyloxyreste für X bestehen dabei bevorzugt aus 1 bis 20 C-Atomen, besonders bevorzugt sind Reste aus der Gruppe enthaltend p-Toluolsulfonyloxy- (Tosyl-), p-Brombenzolsulfonyloxy- und p-Nitrobenzolsulfonyloxy-Reste.

Alkoxyreste für X bestehen dabei bevorzugt aus 1 bis 20 C-Atomen, besonders bevorzugt sind Reste aus der Gruppe enthaltend Methoxy- und Ethoxy-Reste.

Aryloxyreste für X bestehen dabei bevorzugt aus 1 bis 20 C-Atomen, besonders bevorzugt sind Reste aus der Gruppe enthaltend Phenoxy-, 4-Nitrophenoxy- und 2,4,6-Trinitrophenoxy-Reste.

Das als Edukt eingesetzte 2,6-Diaminopurinderivat der allgemeinen Formel (5) enthält mindestens einen Silylrest R¹² am Stickstoffatom in 9-Position, sowie optional weitere Silylreste an den beiden Aminofunktionen in 2- und 6-Position, welche in einer möglichen Ausführungsform in einem Reaktionsschritt zusammen mit der Silylgruppe R¹² am Stickstoffatom in 9-Position eingeführt werden und bei der weiteren Umsetzung nach dem erfindungsgemäßen Verfahren als Aminoschutzgruppen für die beiden Aminofunktionen in 2- und 6-Position fungieren. Ein persilyliertes Edukt der allgemeinen Formel (5) kann dabei bis zu 5 gleiche oder verschiedene Silylreste enthalten. Bevorzugt sind 2,6-Diaminopurinderivate der allgemeinen Formel (5) mit ein bis drei Silylresten, ganz besonders bevorzugt solche mit drei Silylresten, insbesondere mit einem Silylrest am Stickstoff in 9-Position und jeweils einem Silylrest an den beiden Aminofunktionen in 2- und 6-Position.

Die Silylreste für R¹² enthalten dabei in der Regel am Si-Atom aliphatische und/oder aromatische Substituenten mit jeweils 1 bis 20 C-Atomen, insbesondere mit jeweils 1 bis 10 C-Atomen. Bevorzugt sind Reste aus der Gruppe enthaltend Trimethylsilyl-, Triethylsilyl-, Triisopropylsilyl-, *tert*-Butyldimethylsilyl- und *tert*-Butyldiphenylsilyl-Reste. Besonders bevorzugt ist der Trimethylsilyl-Rest.

Die mindestens am Stickstoffatom in 9-Position silylierten 2,6-Diaminopurinderivate der allgemeinen Formel (5) können auch bezüglich der Aminfunktionen in 2- und 6-Position als freie Amine, in ungeschützter Form, oder in einer Form, in der eine oder beide primäre Aminogruppen mit anderen Aminoschutzgruppen geschützt werden, eingesetzt werden.

Die Reste **R⁸, R⁹, R¹⁰, R¹¹** können folglich unabhängig voneinander für Wasserstoff oder eine Aminoschutzgruppe stehen.

Eine Auswahl an geeigneten Aminoschutzgruppen kann der Fachmann aus T.W. Greene, P.G.M. Wuts, "Protective Groups in Organic Synthesis", 2nd Edition, Wiley 1991, S. 309-385 entnehmen. Als Aminoschutzgruppen werden dabei vorzugsweise Acylreste, Acyloxycarbonylreste, Alkylreste, Arylalkylreste oder Silylreste verwendet.

Acylreste als Aminoschutzgruppe leiten sich dabei bevorzugt aus einer aromatischen oder aliphatischen Carbonsäure mit 2 bis 20 C-Atomen ab, besonders bevorzugt mit den Resten aus der Gruppe enthaltend Benzoyl-, Acetyl- und Formyl-Reste.

Acyloxycarbonylreste als Aminoschutzgruppe besitzen dabei vorzugsweise 2 bis 20 C-Atome, besonders bevorzugt sind Reste aus der Gruppe enthaltend tert-Butyloxycarbonyl- (BOC-), 9-Fluorenylmethyloxycarbonyl- (Fmoc-) und Benzyloxycarbonyl-(Z-) Reste.

Alkylreste als Aminoschutzgruppe bestehen dabei bevorzugt aus 1 bis 20 C-Atomen, besonders bevorzugt sind Reste aus der Gruppe enthaltend Methyl- und Allyl-Reste.

Arylalkylreste als Aminoschutzgruppe bestehen dabei bevorzugt aus 1 bis 20 C-Atomen, besonders bevorzugt sind Reste aus der Gruppe enthaltend Benzyl- und 4-Methoxybenzyl-Reste.

Silylreste als Aminoschutzgruppe können dabei am Si-Atom aliphatische und/oder aromatische Substituenten mit jeweils 1 bis 20 C-Atomen, insbesondere mit jeweils 1 bis 10 C-Atomen enthalten. Bevorzugt sind Reste aus der Gruppe enthaltend Trimethylsilyl-, Triethylsilyl-, Triisopropylsilyl-, *tert-*Butyldimethylsilyl- und *tert*-Butyldiphenylsilyl-Reste. Besonders bevorzugt ist der Trimethylsilyl-Rest.

In einer besonders bevorzugten Ausführungsform des erfindungsgemäßen Verfahrens stehen R¹² und jeweils ein Rest R⁸ oder R⁹ bzw. R¹⁰ oder R¹¹ der Aminofunktionen in 2- und 6-Position für Trimethylsilyl und die jeweils anderen Reste R⁸ oder R⁹ bzw. R¹⁰ oder R¹¹ für Wasserstoff.

Die bei der Reaktion anwesende 1,3-Dicarbonylverbindung ist vorzugsweise ein β-Carbonylcarbonsäureester, insbesondere ein β-Ketocarbonsäureester, ein 1,3-Diketon oder ein Malonsäurederivat mit 5 bis 20 C-Atomen gemäß der allgemeinen Formel (3), 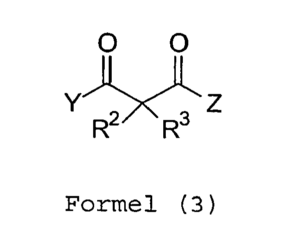 oder ein Silylderivat eines β-Carbonylcarbonsäureesters, insbesondere eines β-Ketocarbonsäureesters eines 1,3-Diketons oder eines Malonsäurederivates gemäß der allgemeinen Formel (4), 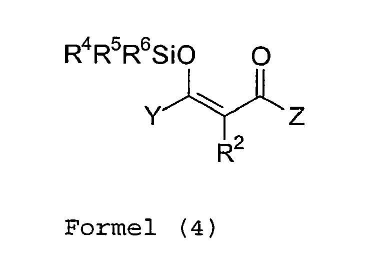 wobei **Y** und **Z** in der allgemeinen Formeln (3) und der allgemeinen Formel 4 unabhängig voneinander Wasserstoff, einen Alkylrest mit 1 bis 20 C-Atomen, einen Arylrest mit 6 bis 20 C-Atomen oder eine Alkyloxygruppe mit 1 bis 20 C-Atomen und
**R²** und **R³** in der allgemeinen Formel (3) unabhängig voneinander und **R²** in der allgemeinen Formel (4) Wasserstoff, einen Acylrest einer aromatischen oder aliphatischen Carbonsäure mit 2 bis 20 C-Atomen, einen Alkylrest mit 1 bis 20 C-Atomen oder einen Arylrest mit 6 bis 20 C-Atomen und
**R⁴, R⁵** und **R⁶** in der allgemeinen Formel (4) unabhängig voneinander einen aliphatischen oder aromatischen Rest mit 1 bis 20 C-Atomen bedeuten können.

Besonders bevorzugt sind 1,3-Dicarbonylverbindungen aus der Gruppe enthaltend Glyoxylsäuremethylester, Glyoxylsäureethylester, Acetessigsäuremethylester, Acetessigsäureethylester, Acetessigsäure-*tert*-butyl-ester, Acetessigsäure-isobutylester, Acetessigsäure-isopropylester, Acetessigsäure-n-propylester, Acetessigsäure-benzylester, 2-Acetyl-acetessigsäuremethylester, 2-Acetylacetessigsäureethylester, 2-Acetyl-acetessigsäure-*tert*-butylester, 3-Oxopentansäuremethylester, 3-Oxopentansäureethylester, 3-Oxopentansäure-*tert*-butylester, 3-Oxohexansäuremethylester, 3-Oxohexansäureethylester, Acetylaceton, 2,4-Hexandion, 3,5-Heptandion, Malonsäuredimethylester, Malonsäurediethylester, Malonsäurediisobutylester, Malonsäurediisopropylester und Malonsäuredi-*tert*-butylester.

Besonders bevorzugt sind Silylderivate von 1,3-Dicarbonylverbindungen aus der Gruppe enthaltend3-Trimethylsilyloxyacrylsäuremethylester, 3-Trimethylsilyloxyacrylsäureethylester, 4-Trimethylsilyloxypent-3-en-2-on, 4-Triethylsilyloxypent-3-en-2-on, 4-(*tert-*Butyldimethylsilyloxy)pent-3-en-2-on, 4-(tert-Butyldiphenylsilyloxy)pent-3-en-2-on, 3-Trimethylsilyloxybut-2-ensäure-methylester, 3-Trimethylsilyloxybut-2-ensäure-ethylester, 3-Trimethylsilyloxybut-2-ensäure-*tert*-butylester.

Als Lewis-Säuren sind bevorzugt Trialkylsilylhalogenide und Perfluoralkansulfonsäuretrialkylsilylester. Besonders bevorzugt sind dabei Iodtrimethylsilan und Trifluormethansulfonsäuretrimethylsilylester. Ganz besonders bevorzugt ist Iodtrimethylsilan.

Als Lösungsmittel können generell alle aprotischen organischen Lösungsmittel verwendet werden. Beispiele für geeignete Lösungsmittel sind Methylenchlorid (Dichlormethan), 1,2-Dichlorethan und Acetonitril. Besonders bevorzugt sind Methylenchlorid (Dichlormethan) und 1,2-Dichlorethan. Ganz besonders bevorzugt ist Methylenchlorid (Dichlormethan).

Die Reaktion kann grundsätzlich bei einer beliebigen Temperatur durchgeführt werden, die in der Regel nur durch die Eigenschaften des Lösungsmittels und der eingesetzten Komponenten (Siedepunkt, Schmelzpunkt, Löslichkeit, etc.) eingeschränkt wird.

Vorzugsweise wird die Reaktion bei einer Temperatur zwischen -5°C und dem Siedepunkt des verwendeten Lösungsmittels durchgeführt. Besonders bevorzugt wird bei einer Temperatur zwischen 0°C und +30°C gearbeitet.

Vorzugsweise wird bei Atmosphärendruck gearbeitet.

Als Zuckerbausteine in Form von Verbindungen der allgemeinen Formel (2) haben sich insbesondere Verbindungen mit einer Abgangsgruppe X = OCOR⁷ (Acyloxyreste) als zweckmäßig erwiesen, wobei sich der Rest -COR⁷ von einer aliphatischen oder aromatischen Carbonsäure mit 1 bis 20 C-Atomen ableitet, insbesondere Essigsäure (Acetoxy-), Propionsäure (Propionyloxy-), n-Buttersäure (n-Butyryloxy-), Trifluoressigsäure (Trifluoracetoxy-) oder Benzoesäure (Benzoyloxy-).

Zur Herstellung solcher Verbindungen stehen eine Reihe von bekannten Verfahren zur Auswahl, wobei sich u.a. die Synthese aus 1,3-Dioxolan-4-on-Derivaten bewährt hat.

Hierbei wird nach WO 92/14729 die Lactonfunktion mit einem selektiven Reduktionsmittel reduziert und das Produkt ohne Isolierung direkt mit einem Acylierungsmittel, wie z.B. Acetanhydrid oder Benzoylchlorid umgesetzt. Als Reduktionsmittel eignen sich z.B. Diisobutylaluminiumhydrid (DIBAL-H) oder Lithium-tri(*tert*-butoxy)aluminiumhydrid (LTTBA).

Die Verbindungen vom Typ der allgemeinen Formel (2) sind in den so dargestellten Rohprodukten mit Gehalten von ca. 40 bis 70% enthalten. Als Nebenprodukte und weitere Bestandteile der Rohprodukte können dabei unter anderem 1,3-Dicarbonylverbindungen, insbesondere β-Ketocarbonsäureester wie z.B. Acetessigsäure-*tert*-butylester oder Acetessigsäureisobutylester identifiziert werden.

Es hat sich als besonders vorteilhaft herausgestellt, die nach WO 92/14729 hergestellten Ausgangsverbindungen der allgemeinen Formel 2 der Silyl-Hilbert-Johnson-Reaktion ohne vorherige Abtrennung der enthaltenen 1,3-Dicarbonylverbindungen aus den Rohprodukten zuzuführen. Die der Reaktionsmischung auf diese Weise zugeführten 1,3-Dicarbonylverbindungen können so bei der Durchführung des erfindungsgemäßen Verfahrens, der Umsetzung von Verbindungen vom Typ der allgemeinen Formel 2 mit silylierten 2,6-Diaminopurinderivaten der allgemeinen Formel 5 in Gegenwart von Lewis-Säuren direkt den erfinderischen Effekt bewirken.

Der Vergleichsversuch belegt, dass, wenn das nach WO 92/14729 hergestellte Edukt der allgemeinen Formel (2) beispielsweise durch Chromatographie von 1,3-Dicarbonylverbindungen befreit wird (Beispiel 4), in der nachfolgenden Umsetzung mit dem silyliertem 2,6-Diaminopurin der allgemeinen Formel (5) nur eine sehr geringe Ausbeute des gewünschten Produktes der allgemeinen Formel 1 im Rahmen eines praktisch nicht auftrennbaren komplexen Produktgemisches erhalten wird (Beispiel 5).

Durch den gezielten, erfindungsgemäßen Zusatz zu der gereinigten Verbindung vom Typ der allgemeinen Formel (2) von 1,3-Dicarbonylverbindungen oder deren silylierten Derivaten wird hingegen das gewünschte Produkt mit einer hohen Ausbeute und Reinheit erhalten.

Nach dem erfindungsgemäßen Verfahren können 1,3-Dicarbonylverbindungen bzw. deren silylierte Derivate entweder durch gezielten Zusatz oder durch Auswahl einer entsprechenden Synthese des Ausgangsmaterials der allgemeinen Formel (2) der Reaktionsmischung für die Glykosylierung zugeführt werden und der erfindungsgemäße Effekt erzielt werden.

Bei der beschriebenen Silyl-Hilbert-Johnson-Reaktion entstehen immer zwei Hauptprodukte, die sich durch ihre relative Stereochemie unterscheiden. Wie bereits in WO 97/21706 beschrieben, ist aufgrund der Abwesenheit eines 2'-Substituenten (Zucker-Zählweise) die Stereoselektivität (cis / trans) nur sehr schwach ausgeprägt.

Im Gegensatz zur Lehre der WO 97/21706 konnte bei der erfindungsgemäßen Umsetzung von Verbindungen vom Typ der allgemeinen Formel (2) mit silyliertem 2,6-Diaminopurin keine Abhängigkeit der cis/trans-Selektivität von der Reaktionstemperatur beobachtet werden. Variiert man die Temperatur in einem Bereich zwischen -78°C und +25°C, so ist das gefundene Isomerenverhältnis im Rahmen nicht signifikanter Schwankungen konstant.

Überraschenderweise konnte jedoch eine signifikante Auswirkung auf die cis/trans-Selektivität in Abhängigkeit von der verwendeten Lewis-Säure beobachtet werden (vgl. Tabelle 2): Während bei Verwendung von Iodtrimethylsilan in der Regel das cis-Isomere das Hauptprodukt darstellt (cis/trans-Verhältnis zwischen 1.5:1 und 2.5:1), wurde bei Verwendung von Trifluormethansulfonsäuretrimethylsilylester als Hauptprodukt das trans-Isomer gefunden (cis/trans-Verhältnis zwischen 0.7:1 und 0.8:1).

Die Aufarbeitung der Reaktionsmischungen nach dem erfindungsgemäßen Verfahren erfolgt zweckmäßigerweise zunächst durch Hydrolyse mit Wasser, wässriger Säure oder Lauge. Dabei werden sowohl die verwendete Lewis-Säure und in Abhängigkeit von den Aufarbeitungsbedingungen gegebenenfalls auch gleichzeitig die Aminoschutzgruppen der gebildeten Reaktionsprodukte hydrolysiert, so dass in 2- und 6- Position freie Aminfunktionen resultieren. In Abhängigkeit des gewünschten Endproduktes, können die Hydrolysebedingungen auch so gewählt werden, dass die Aminoschutzgruppen in 2- und 6-Position erhalten bleiben.

Silylschutzgruppen, wie z.B. Trimethylsilyl-, werden bereits durch verdünnte Säure bei Zimmertemperatur abgespalten, während Acylreste wie z.B. Benzoyl- erst durch Erhitzen im Basischen oder durch Umsetzung mit Ammoniak bzw. Aminen gespalten werden.

Typischerweise wird die hydrolytische Aufarbeitung zunächst im sauren pH-Bereich (pH 0 bis 3) vorgenommen. Nebenprodukte und Verunreinigungen können durch Extraktion mit einem organischen Lösungsmittel, insbesondere Methylenchlorid, 1,2-Dichlorethan, Toluol, Hexan, Heptan, THF oder Diethylether entfernt werden. Anschließend wird durch Zugabe von wässriger Lauge, insbesondere wässriger Lösungen von Natriumhydroxid, Natriumcarbonat, Natriumhydrogencarbonat oder Kaliumcarbonat und erneute Extraktion mit einem organischen Lösungsmittel, insbesondere den oben genannten Lösungsmitteln, das Produkt in die organische Phase überführt.
Nach dem Entfernen des organischen Lösungsmittels durch Destillation wird das Produkt erhalten, das anschließend ggf. noch durch weitere Schritte, insbesondere Umkristallisation oder Chromatographie weiter gereinigt werden kann.
Die abschließende wässrige Hydrolyse führt demzufolge zu Reaktionsprodukten der allgemeinen Formel (1) in denen die Reste **R⁸, R⁹, R¹⁰** und **R¹¹** unabhängig voneinander für Wasserstoff oder eine Aminoschutzgruppe stehen können, insbesondere zu solchen Reaktionsprodukten der allgemeinen Formel (1) in denen alle Reste **R⁸, R⁹, R¹⁰** und **R¹¹** für Wasserstoff stehen.

In einer bevorzugten Ausführungsform des erfindungsgemäßen Verfahrens lassen sich optisch reine Reaktionsprodukte - durch Auswahl entsprechend optisch konfigurierter Edukte - in den optischen Konfigurationen der allgemeinen Formeln (1a), (1b), (1c) und (1d) herstellen, in denen alle Reste **R⁸, R⁹, R¹⁰** und **R¹¹** für Wasserstoff stehen. Ganz besonders bevorzugt lässt sich das Verfahren auf die Herstellung von Produkten in der optischen Konfiguration der allgemeinen Formel 1a anwenden, wobei alle Reste **R⁸, R⁹, R¹⁰** und **R¹¹** für Wasserstoff stehen.

Bei Aufarbeitung der nach dem erfindungsgemäßen Verfahren hergestellten Verbindungen kann eine weitere Anreicherung des gewünschten Isomeren (in der Regel das cis-Isomer in der optischen Konfiguration der allgemeinen Formeln 1a oder 1c) insbesondere durch Kristallisation oder Säulenchromatographie erreicht werden. Dabei ist insbesondere bei der großtechnischen Umsetzung des erfindungsgemäßen Verfahrens die Kristallisation aus Kostengründen vorzuziehen.
So ist ein weiterer Gegenstand der Erfindung ein Verfahren zur Reinigung der nach dem erfindungsgemäßen Verfahren erhaltenen Verbindungen der allgemeinen Formel (1) durch Umkristallisation.

Da die Produkte der allgemeinen Formel (1) stark polare Verbindungen mit relativ hohen Schmelzpunkten darstellen, eignen sich insbesondere polare Lösungsmittel wie Alkohole, Ether oder Ester mit 1-10 C-Atomen für die Umkristallisation.

So ist beispielsweise für die Umkristallisation von Isobutyraten der allgemeinen Formel (1) wobei R¹ = (H₃C)₂CHCO-bedeutet, Isopropanol als Lösungsmittel besonders bevorzugt.

Durch einmalige Kristallisation wird aus dem Gemisch (cis /trans ~ 60-70 : 30-40) ein Produkt erhalten, das zwischen 97 und 99% Gehalt an cis-Isomer aufweist (vgl. Beispiel 4). Durch erneute Umkristallisation kann bis auf Gehalte von >99.5% des cis-Isomers angereichert werden.

Zur Herstellung von (nicht OH-geschützten) [4-(2,6-diamino-9H-purin-9-yl)-1,3-dioxolan-2-yl]methanol-Derivaten der allgemeinen Formel 1, wobei R¹ hier Wasserstoff bedeutet, werden dem Fachmann geläufige Verfahren zur Abspaltung der entsprechenden OH-Schutzgruppe angewandt.

Eine Auswahl von Verfahren ist insbesondere in T.W. Greene, P.G.M. Wuts, "Protective Groups in Organic Synthesis", 2nd Edition, Wiley 1991, S. 10-117 beschrieben.

So ist ein weiterer Gegenstand der Erfindung die Verwendung der nach dem erfindungsgemäßen Verfahren erhaltenen Verbindungen der allgemeinen Formel (1) zur Herstellung von Verbindungen der allgemeinen Formel (6)

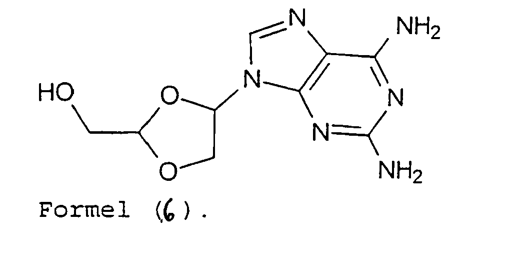

Dies erfolgt allgemein durch die Abspaltung der Hydroxylschutzgruppen.

In einer besonders bevorzugten Variante werden bereits Verbindungen der allgemeinen Formel (1) eingesetzt, in denen die Aminoschutzgruppen bereits nach dem oben beschriebenen Verfahren abgespalten wurden und demnach alle Reste **R⁸, R⁹, R¹⁰** und **R¹¹** für Wasserstoff stehen.

Die Verbindungen der allgemeinen Formel (6) können in racemischer Form oder in optisch reiner Form erhalten werden. Insbesondere lassen sich die erfindungsgemäß hergestellten Produkte der allgemeinen Formel (1) in optisch reiner Form zu optisch reinen Verbindungen der allgemeinen Formel (6) umsetzen.

In einer besonders bevorzugten Verwendung der erfindungsgemäß hergestellten Produkte kann auf diese Weise (*2R,4R*)-[4-(2,6-diamino-9*H*-purin-9-yl)-1,3-dioxolan-2-yl]methanol [(-)-DAPD] hergestellt werden.

Bevorzugte Verfahren zur Abspaltung von Acylresten als OH-Schutzgruppe sind die Umsetzung mit Ammoniak oder aliphatischen Aminen, die basische wässrige Hydrolyse und die Umsetzung mit Alkoholaten wie z.B. Natriummethylat.

### Beispiele

(Der in einigen Beispielen vorkommende griechische Buchstabe "Ξ" bezeichnet ein Stereozentrum mit nicht festgelegter absoluter Konfiguration)

### Beispiel 1: (Silylierung von 2,6-Diaminopurin)

75 g 2,6-Diaminopurin, 17,8 g Ammoniumsulfat und 1451 g Hexamethyldisilazan wurden in einem 4-1-Dreihalskolben vorgelegt. Die Suspension wurde unter Rühren zum Rückfluss erhitzt (Rückflussbeginn 108°C) und dort für 3-4 h gehalten, wobei die Rückflusstemp. auf 122°C ansteigt und die Mischung klar wurde. Die Lösung wurde etwas abgekühlt (auf ca. 80°C) und langsam Vakuum angezogen. Das überschüssige Hexamethyldisilazan wurde anschließend bis 85°C Sumpftemp. / 5 mbar abdestilliert. Die GC-Analyse des Rückstandes zeigte folgende Zusammensetzung: 86.3% Tris(trimethylsilyl)-2,6-diaminopurin, 0.5% Trimethylsilyl-2,6-diaminopurin, 0.9% Bis(trimethylsilyl)-2,6-diaminopurin, 10.0% Bis(trimethylsilyl)sulfat, 0.3% Hexamethyldisilazan.
Der Rückstand wurde in trockenem Methylenchlorid gelöst. Die Menge wurde dabei so berechnet, dass eine ca. 1-molare Lösung entstand.

### Beispiel 2: (Herstellung von (2R-4Ξ)-4-Acetoxy-2-isobutyryloxymethyl-1,3-dioxolan)

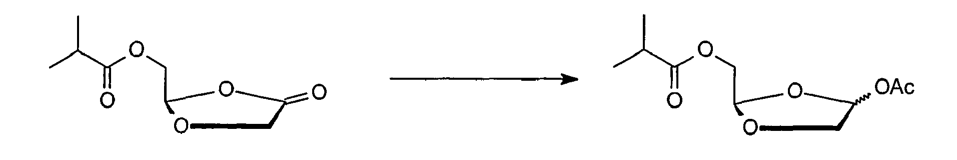

316,2 g einer 1.1-molaren Lösung von LiAlH(OtBu)₃ in THF und 142 g THF wurden in einem trockenem Kolben vorgelegt und auf - 5°C abgekühlt. 53.0 g (2R)-2-Isobutyryloxymethyl-1,3-dioxolan-4-on wurden innerhalb von 45 min zugetropft. Die Mischung wurde auf 25°C erwärmt, 33,7 g 4-Dimethylaminopyridin zugegeben und 1 h gerührt. Es wurde erneut auf -5°C abgekühlt und anschließend 209 g Acetanhydrid bei dieser Temperatur zugetropft. Die Mischung wurde 15 h bei -5 bis 0°C gerührt.
Dann wurde durch Zugabe von 415 g 15%iger NH₄Cl-Lösung gequencht und mit 400 g Wasser verdünnt. Das THF wurde durch Abdestillieren im Vakuum weitgehend entfernt und anschließend 480 g Ethylacetat zugegeben. Die Mischung wurde gut durchgeschüttelt und die Phasen wurden getrennt. Die wässrige Phase wurde einmal mit Ethylacetat nachextrahiert und die vereinigten organischen Phasen mit Wasser und NaHCO₃-Lösung gewaschen. Das Lösungsmittel wurde im Vakuum abdestilliert und man erhielt das Produkt als orangefarbene Flüssigkeit (Ausbeute 69.0 g mit 65% Gehalt entspr. 70% d.Th.).
Das Produkt wird nach diesem Verfahren mit Gehalten zwischen 40 und 70% (GC) als cis/trans-Gemisch erhalten. Das cis/trans-Verhältnis beträgt zwischen 2.8 und 3.6. Als weitere Bestandteile sind u.a. enthalten Acetessigsäure-*tert*-butylester (ca. 8 bis ca. 27%) und 2-Acetyl-acetessigsäure-*tert*-butylester (ca. 6% bis ca. 15%).

### Beispiel 3: (Reinigung von (2R-4Ξ)-4-Acetoxy-2-isobutyryloxymethyl-1,3-dioxolan durch Säulenchromatographie)

30 g eines nach Beispiel 2 hergestellten Produktes mit einem Gehalt von 42.5% wurden über 200 g Kieselgel chromatographiert (Laufmittel n-Heptan / Ethylacetat 4:1). Die produkthaltigen Fraktionen wurden vereinigt und das Lösungsmittel im Vakuum entfernt. Man erhielt 14 g farblose Flüssigkeit. Der Gehalt nach GC betrug 79.5% (Ausbeute 88%).

### Beispiel 4: (Herstellung von cis- und trans- (2R)-2-Isobutyryloxymethyl-4-(2,6-diaminopurin-9-yl)-1,3-dioxolan)

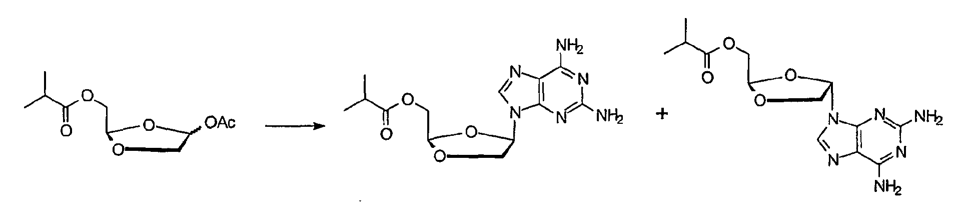

105.1 g (*2R*-*4*-*Ξ*)-4-Acetoxy-2-isobutyryloxymethyl-1,3-dioxolan mit einem Gehalt von 55.3% (13.2% Acetessigsäure-*tert-*butylester und 6.3% 2-Acetyl-acetessigsäure-*tert*-butylester; dies entspricht 0.48 Mol an 1,3-Dicarbonylverbindung pro Mol Dioxolan) wurden in 540 ml Methylenchlorid vorgelegt. Eine Lösung von 19.8 g Acetessigsäure-*tert*-butylester in 120 ml Methylenchlorid und 275 ml einer 1.0-molaren Lösung von Tris(trimethylsilyl)-2,6-diaminopurin in Methylenchlorid (nach Beispiel 1) wurden ebenfalls zugegeben. Die Lösung wurde auf 0°C abgekühlt und bei dieser Temperatur eine Lösung von 125.0 g Iodtrimethylsilan (Gehalt 95.8%) in 250 ml Methylenchlorid innerhalb von 20 min zugetropft. Die Mischung wurde 1 h bei 0°C nachgerührt, auf 25°C erwärmt und bei dieser Temperatur 15 h gerührt. Durch Zugabe von 1 1 einer 10%igen Na₂CO₃-Lösung wurde gequencht und 10 min gerührt. Die Phasen wurden getrennt und die wässrige Phase zweimal mit Methylenchlorid nachextrahiert. Die vereinigten organischen Phasen wurden mit 10%iger Na₂CO₃-Lösung und Wasser gewaschen und über Kieselgur filtriert. Die Lösung wurde anschließend auf ca. 1000 ml aufkonzentriert. Die HPLC-Analyse der Lösung zeigte 67.8% cis-(*2R*)-2-Isobutyryloxymethyl-4-(2,6-diaminopurin-9-yl)-1,3-dioxolan und 31.7% trans-(*2R*)-2-Isobutyryloxymethyl-4-(2,6-diaminopurin-9-yl)-1,3-dioxolan (Isomerenverhältnis 2.1:1; HPLC Fl%). Die Lösung wurde mit 750 ml 0.5N HCl versetzt und 30 min gerührt. Die Phasen wurden getrennt und die wässrige Phase viermal mit Methylenchlorid gewaschen.
Nach erneuter Zugabe von 500 ml Methylenchlorid wurde der pH-Wert durch Zugabe von 10%iger Na₂CO₃-Lösung auf 8-9 eingestellt und 15 min gerührt.

Die Phasen wurden getrennt und die wässrige Phase mit Methylenchlorid nachextrahiert.
Das Methylenchlorid wurde weitgehend abdestilliert und 1 1 Isopropanol zugegeben. Man destillierte weiter, bis eine Kopftemperatur von 80°C erreicht war. Ausgefallenes Produkt wurde durch Zugabe von weiteren 900 ml Isopropanol in der Siedehitze gelöst. Die Lösung wurde innerhalb von 2 h auf 25°C abgekühlt, 1 h nachgerührt und die ausgefallenen Kristalle abgesaugt. Anschließend wurde mit Isopropanol gewaschen und bei 60°C im Vakuum getrocknet.
Man erhielt 35.2 g farblose Kristalle, die laut NMR-Analyse neben (*2R*)-2-Isobutyryloxymethyl-4-(2,6-diaminopurin-9-yl)-1,3-dioxolan noch 1 Mol Isopropanol enthielten. Nach HPLC waren 98.0% cis- und 1.6% trans-(2R)-2-Isobutyryloxymethyl-4-(2,6-diaminopurin-9-yl)-1,3-dioxolan enthalten. Dies entspricht einer Ausbeute von 36.1% cis-Isomer bezogen auf (*2R*-*4Ξ*)-4-Acetoxy-2-isobutyryloxymethyl-1,3-dioxolan.
Durch Umkristallisation aus Isopropanol erhielt man reines cis-Isomer mit einem Gehalt von 99.6% (Umkristallisationsausbeute 90%) .

### Beispiel 5: (Vergleichsbeispiel)

9.3 ml einer 1.0-molaren Lösung von Tris(trimethylsilyl)-2,6-diaminopurin in Methylenchlorid (nach Beispiel 1) wurden auf 0°C abgekühlt. Die Lösung von 2.52 g (*2R*-*4Ξ*)-4-Acetoxy-2-isobutyryloxymethyl-1,3-dioxolan aus Beispiel 3 mit einem Gehalt von 79.5% wurde zugegeben. Bei 0 bis 5°C wurde eine Lösung von 4.0 g Iodtrimethylsilan zugegeben, anschließend auf 25°C erwärmt und 15 h gerührt. Es wurde mit 10%iger Na₂CO₃-Lösung hydrolysiert. Die Phasen wurden anschließend getrennt und die organische Phase wird im HPLC analysiert.
Das HPLC der organischen Phase zeigte neben einer Vielzahl von Nebenprodukten 1.6% 2,6-Diaminopurin sowie 34.2% cis- und 30.4% trans-(2R)-2-Isobutyryloxymethyl-4-(2,6-diaminopurin-9-yl)-1,3-dioxolan.

### Beispiel 6:

Es wurde analog zu Beispiel 5 vorgegangen, mit dem Unterschied, dass vor der Zugabe des Iodtrimethylsilans eine Lösung von 1.16 g Acetessigsäure-*tert*-butylester in 5 ml Methylenchlorid zugegeben wurde.
Das HPLC zeigte kein 2,6-Diaminopurin sowie 67.8% cis- und 31.7% trans-(*2R*)-2-Isobutyryloxymethyl-4-(2,6-diaminopurin-9-yl)-1,3-dioxolan.

### Beispiel 7:

Es wurde analog zu Beispiel 5 vorgegangen, mit dem Unterschied, dass vor der Zugabe des Iodtrimethylsilans eine Lösung von 0.9 g 3-Trimethylsilyloxy-2-butensäure-*tert*-butylester in 5 ml Methylenchlorid zugegeben wurde.
Das HPLC zeigte 0.5% 2,6-Diaminopurin sowie 62.6% cis- und 33.6% trans-(2*R*)-2-Isobutyryloxymethyl-4-(2,6-diaminopurin-9-yl)-1,3-dioxolan.

### Beispiel 8:

10,3 ml einer 0.93-molaren Lösung von Tris(trimethylsilyl)-2,6-diaminopurin in Methylenchlorid (nach Beispiel 1) wurden in einem trockenem Kolben vorgelegt.
3.97 g (*2R*-*4Ξ*)-4-Acetoxy-2-isobutyryloxymethyl-1,3-dioxolan (vgl. Beispiel 2) mit einem Gehalt von 50.4% und einem Gehalt von 17.1% Acetessigsäure-*tert*-butylester sowie 7.5% 2-Acetyl-acetessigsäure-*tert*-butylester (dies entspricht 0.67 Mol an 1,3-Dicarbonylverbindung pro Mol Dioxolan) wurden in 20 ml Methylenchlorid gelöst und zugegeben. Bei 0°C wurde eine Lösung von 4.31 g Iodtrimethylsilan in 10 ml Methylenchlorid zugegeben. Die Mischung wurde auf 25°C erwärmt und 15 h gerührt. Anschließend wurde mit 10%iger Na₂CO₃-Lösung hydrolysiert.
Die Phasen wurden getrennt und die organische Phase wird im HPLC analysiert.

Das HPLC zeigte kein 2,6-Diaminopurin sowie 58.2% cis- und 28.0% trans-(*2R*)-2-Isobutyryloxymethyl-4-(2,6-diaminopurin-9-yl)-1,3-dioxolan.

### Beispiel 9:

Es wurde analog zu Beispiel 8 vorgegangen unter Verwendung von 3.40 g (*2R*-*4Ξ*)-4-Acetoxy-2-isobutyryloxymethyl-1,3-dioxolan mit einem Gehalt von 58.9% (9.0% Acetessigsäure-*tert*-butylester und 8.7% 2-Acetyl-acetessigsäure-*tert*-butylester; dies entspricht 0.40 Mol an 1,3-Dicarbonylverbindung pro Mol Dioxolan) mit dem Unterschied, dass statt Acetessigsäure-*tert*-butylester 0.5 Mol-Eq Acetessigsäuremethylester zugegeben wurde.
Das HPLC zeigte kein 2,6-Diaminopurin sowie 59.5% cis- und 27.1% trans-(*2R*)-2-Isobutyryloxymethyl-4-(2,6-diaminopurin-9-yl)-1,3-dioxolan.

### Beispiel 10:

Es wurde analog zu Beispiel 9 vorgegangen mit dem Unterschied, dass kein weiterer Zusatz einer 1,3-Dicarbonylverbindung vorgenommen wurde (d.h. nur der aus dem Edukt stammende Anteil von 0.4 Mol 1,3-Dicarbonylverbindung pro Mol Dioxolan enthalten ist).
Das HPLC zeigte 8.9% 2,6-Diaminopurin sowie 45.9% cis- und 28.8% trans-(*2R*)-2-Isobutyryloxymethyl-4-(2,6-diaminopurin-9-yl)-1,3-dioxolan.

### Beispiel 11:

Es wurde analog zu Beispiel 4 vorgegangen unter Verwendung von 49.3 g (*2R*-*4Ξ*)-4-Acetoxy-2-isobutyryloxymethyl-1,3-dioxolan mit einem Gehalt von 58.9% (9.0% Acetessigsäure-*tert*-butylester und 8.7% 2-Acetyl-acetessigsäure-*tert*-butylester; dies entspricht 0.40 Mol an 1,3-Dicarbonylverbindung pro Mol Dioxolan) mit dem Unterschied, dass statt Acetessigsäure-*tert*-butylester 0.5 Mol-Eq Acetylaceton zugegeben wurden.

Die HPLC-Analyse des Rohproduktes zeigte 57.1% cis-(2R)-2-Isobutyryloxymethyl-4-(2,6-diaminopurin-9-yl)-1,3-dioxolan und 27.1% trans-(*2R*)-2-Isobutyryloxymethyl-4-(2,6-diaminopurin-9-yl)-1,3-dioxolan (Isomerenverhältnis 2.1:1; HPLC F1%).
Nach Kristallisation aus Isopropanol wurden 17.7 g farblose Kristalle erhalten, die laut NMR-Analyse neben (*2R*)-2-Isobutyryloxymethyl-4-(2,6-diaminopurin-9-yl)-1,3-dioxolan noch 1 Mol Isopropanol enthielten. Nach HPLC waren 97.5% cis- und 2.2% trans-(*2R*)-2-Isobutyryloxymethyl-4-(2,6-diaminopurin-9-yl)-1,3-dioxolan enthalten. Dies entspricht einer Ausbeute von 36.1% cis-Isomer bezogen auf (*2R*-*4Ξ*)-4-Acetoxy-2-isobutyryloxymethyl-1,3-dioxolan.
Durch Umkristallisation aus Isopropanol erhielt man reines cis-Isomer mit einem Gehalt von 99.8% (Umkristallisationsausbeute 90%) .

**Tabelle 1: Hilbert-Johnson-Reaktion in Gegenwart verschiedener 1,3-Dicarbonylverbindungen (alle Reaktionen in Methylenchlorid in Ggw. von 2.5 Mol-Eq Iodtrimethylsilan):**

| Beispiel | ACTB + AACTB enthalten in Dioxolan-acetat [Mol-Eq] | Zusatz | Mol-Eq | cis-Isomer [Fl%] | trans-Isomer [Fl%] |
|---|---|---|---|---|---|
| 5 | 0.045 | - | - | 34.2 | 30.4 |
| 6 | 0.045 | ACTB | 0.85 | 67.8 | 31.7 |
| 7 | 0.045 | TMS-ACTB | 0.50 | 62.6 | 33.6 |
| 8 | 0.67 | - | - | 58.2 | 28.0 |
| 9 | 0.40 | ACM | 0.5 | 59.5 | 27.1 |
| 10 | 0.4 | - | - | 45.9 | 28.8 |
| 11 | 0.4 | AcAc | 0.5 | 57.1 | 27.1 |

| | | | | | |
|---|---|---|---|---|---|
| ACTB = Acetessigsäure-*tert*-butylester; AACTB = 2-Acetyl-acetessigsäure-*tert*-butylester TMS-ACTB = 3-Trimethylsilyloxybut-2-ensäure-*tert*-butylester | | | | | |

### Beispiel 12:

Es wurde analog zu Beispiel 8 vorgegangen mit dem Unterschied, dass statt Methylenchlorid 1,2-Dichlorethan als Lösungsmittel verwendet wurde.
Das HPLC zeigte kein 2,6-Diaminopurin sowie 53.1% cis- und 29.9% trans-(*2R*)-2-Isobutyryloxymethyl-4-(2,6-diaminopurin-9-yl)-1,3-dioxolan.

### Beispiel 13:

Es wurde analog zu Beispiel 8 vorgegangen mit dem Unterschied, dass statt Methylenchlorid Acetonitril als Lösungsmittel verwendet wurde. Bei der Aufarbeitung wurde Methylenchlorid zugegeben, um das Produkt zu extrahieren.
Das HPLC zeigte kein 2,6-Diaminopurin sowie 45.3% cis- und 36.4% trans-(*2R*)-2-Isobutyryloxymethyl-4-(2,6-diaminopurin-9-yl)-1,3-dioxolan.

### Beispiel 14:

Es wurde analog zu Beispiel 8 vorgegangen mit dem Unterschied, dass statt Iodtrimethylsilan Trifluormethansulfonsäuretrimethylsilylester als Lewis-Säure verwendet wurde.
Das HPLC zeigte kein 2,6-Diaminopurin sowie 33.7% cis- und 47.5% trans-(*2R*)-2-Isobutyryloxymethyl-4-(2,6-diaminopurin-9-yl)-1,3-dioxolan.

### Beispiel 15:

Es wurde analog zu Beispiel 14 vorgegangen mit dem Unterschied, dass an Stelle von Methylenchlorid 1,2-Dichlorethan als Lösungsmittel verwendet wurde.
Das HPLC zeigte kein 2,6-Diaminopurin sowie 31.7% cis- und 40.9% trans-(*2R*)-2-Isobutyryloxymethyl-4-(2,6-diaminopurin-9-yl)-1,3-dioxolan.

**Tabelle 2: Hilbert-Johnson-Reaktion in verschiedenen Lösungsmitteln und mit verschiedenen Lewis-Säuren:**

| Beispiel | ACTB + AACTB enthalten in Dioxolan-acetat [Mol-Eq] | Lösungsmittel | Lewis-Säure [2.5 Mol-Eq] | cis-Isomer [Fl%] | trans-Isomer [Fl%] |
|---|---|---|---|---|---|
| 12 | 0.67 | 1,2-DCE | TMSI | 53.1 | 29.9 |
| 13 | 0.67 | MeCN | TMSI | 45.3 | 36.4 |
| 14 | 0.67 | CH₂Cl₂ | TMSOTf | 33.7 | 47.5 |
| 15 | 0.67 | 1,2-DCE | TMSOTf | 31.7 | 40.9 |

| | | | | | |
|---|---|---|---|---|---|
| ACTB = Acetessigsäure-*tert*-butylester; AACTB = 2-Acetyl-acetessigsäure-*tert*-butylester 1,2-DCE = 1,2-Dichlorethan MeCN = Acetonitril TMSOTf = Trifluormethansulfonsäuretrimethylsilylester (Trimethylsilyltriflat) | | | | | |

### Beispiel 16: (Herstellung von cis- und trans- (2R)-2-Isobutyryloxymethyl-4-(2,6-diaminopurin-9-yl)-1,3-dioxolan)

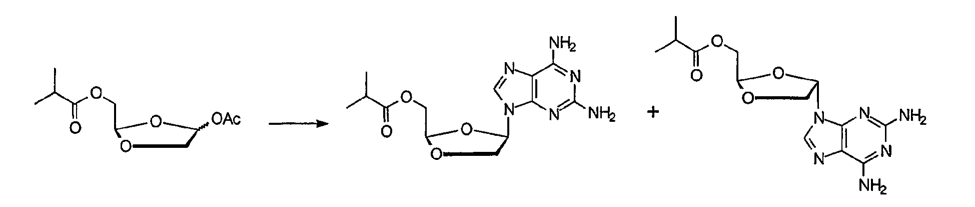

48.0 g (*2R*-*4Ξ*)-4-Acetoxy-2-isobutyryloxymethyl-1,3-dioxolan mit einem Gehalt von 60.5% (9.0% Acetessigsäure-*tert*-butylester und 3.1% 2-Acetyl-acetessigsäure-*tert*-butylester; dies entspricht 0.28 Mol an 1,3-Dicarbonylverbindung pro Mol Dioxolan) wurden in 520 ml Methylenchlorid vorgelegt. 14.8 g Acetessigsäure-*tert*-butylester und 179.8 g einer 0.8-molalen Lösung von Tris(trimethylsilyl)-2,6-diaminopurin in Methylenchlorid (nach Beispiel 1) wurden zugegeben. Die Lösung wurde auf 0°C abgekühlt und bei dieser Temperatur eine Lösung von 62.6 g Iodtrimethylsilan (Gehalt 98%) in 125 ml Methylenchlorid innerhalb von 20 min zugetropft. Die Mischung wurde 20 h bei 0 bis 10°C nachgerührt.
Die Reaktionsmischung wurde bei 0 bis 10°C zu einer Lösung von 34.2 g 20%iger Salzsäure in 725 g Wasser zugetropft. Die Mischung wurde auf 25°C erwärmt und 15 min gerührt.
Die Phasen wurden getrennt und die organische Phase einmal mit 125 ml 0.5N Salzsäure nachextrahiert. Die vereinigten wässrigen Phasen wurden zweimal mit je 100 ml Methylenchlorid gewaschen. Anschließend wurde nach Zugabe von weiteren 474 ml Methylenchlorid der pH-Wert durch Zugabe von 700 g einer 10%igen Natriumcarbonatlösung auf 9.0 eingestellt.
Man rührte 1 h bei 25°C und trennte die Phasen. Die wässrige Phase wurde zweimal mit je 123 ml Methylenchlorid nachextrahiert und die vereinigten organischen Phasen einmal mit 200 ml Wasser gewaschen.
Nach Entfernen des Lösungsmittels im Vakuum erhielt man 27.6 g gelblichen Feststoff.
Die HPLC-Analyse zeigte 71.2% cis-(2R)-2-Isobutyryloxymethyl-4-(2,6-diaminopurin-9-yl)-1,3-dioxolan und 26.5% trans-(*2R*)-2-Isobutyryloxymethyl-4-(2,6-diaminopurin-9-yl)-1,3-dioxolan (Isomerenverhältnis 2.7:1; HPLC Fl%; Rohausbeute 49%).
Das Rohprodukt wurde aus Isopropanol umkristallisiert.
Man erhielt 16.2 g farblose Kristalle, die laut NMR-Analyse neben (*2R*)-2-Isobutyryloxymethyl-4-(2,6-diaminopurin-9-yl)-1,3-dioxolan noch 1 Mol Isopropanol enthielten. Nach HPLC waren 99.0% cis- und 0.9% trans-(*2R*)-2-Isobutyryloxymethyl-4-(2,6-diaminopurin-9-yl)-1,3-dioxolan enthalten. Dies entspricht einer Ausbeute von 33.3% cis-Isomer bezogen auf (*2R*-*4Ξ*)-4-Acetoxy-2-isobutyryloxymethyl-1,3-dioxolan.

### Beispiel 17: (Herstellung von (2R-4Ξ)-4-Acetoxy-2-tert-butyldiphenylsilyloxymethyl-1,3-dioxolan)

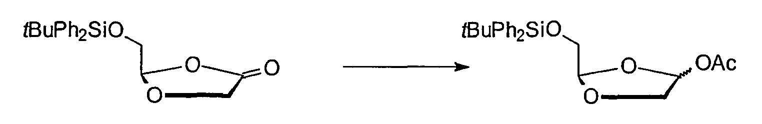

98.4 g LiAlH(OtBu)₃ und 964 g THF wurden in einem trockenem Kolben vorgelegt und auf -10°C abgekühlt. 98.5 g (*2R*)-2-*tert-*Butyldiphenylsilyloxymethyl-1,3-dioxolan-4-on wurden innerhalb von 45 min zugetropft. Die Mischung wurde auf 25°C erwärmt, 33.7g 4-Dimethylaminopyridin zugegeben und 1 h gerührt. Es wurde erneut auf -10°C abgekühlt und anschließend 209 g Acetanhydrid bei dieser Temperatur zugetropft. Die Mischung wurde 15 h bei -10 bis 0°C gerührt.
Dann wurde durch Zugabe von 415 g 15%iger NH₄Cl-Lösung gequencht und mit 400 g Wasser verdünnt. Das THF wurde durch Abdestillieren im Vakuum weitgehend entfernt und anschließend 480 g Ethylacetat zugegeben. Die Mischung wurde gut durchgeschüttelt und die Phasen wurden getrennt. Die wässrige Phase wurde einmal mit Ethylacetat nachextrahiert und die vereinigten organischen Phasen mit Wasser und NaHCO₃-Lösung gewaschen. Das Lösungsmittel wurde im Vakuum abdestilliert und man erhielt das Produkt als orangefarbenes Öl (Ausbeute 110.7 g mit 70% Gehalt entspr. 70% d.Th.).

### Beispiel 18: (Herstellung von cis- und trans- (2R)-2-tert-Butyldiphenylsilyloxymethyl-4-(2,6-diaminopurin-9-yl)-1,3-dioxolan)

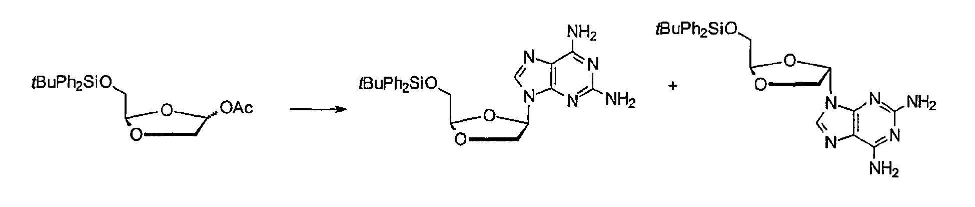

27.5 g (*2R*-*4Ξ*)-4-Acetoxy-2-*tert*-butyldiphenylsilyloxymethyl-1,3-dioxolan mit einem Gehalt von 70.0% (11.2% Acetessigsäure-*tert*-butylester und 5.3% 2-Acetyl-acetessigsäure-*tert-*butylester; dies entspricht 0.50 Mol an 1,3-Dicarbonylverbindung pro Mol Dioxolan) wurden in 96 ml Methylenchlorid vorgelegt. Eine Lösung von 5.4 g Acetessigsäure-*tert*-butylester in 21 ml Methylenchlorid und 50 ml einer 1.0-molaren Lösung von Tris(trimethylsilyl)-2,6-diaminopurin in Methylenchlorid (s. Beispiel 1) wurden ebenfalls zugegeben. Die Lösung wurde auf 0°C abgekühlt und bei dieser Temperatur eine Lösung von 22.6 g Iodtrimethylsilan (Gehalt 95.8%) in 45 ml Methylenchlorid innerhalb von 20 min zugetropft. Die Mischung wurde 1 h bei 0°C nachgerührt, auf 25°C erwärmt und bei dieser Temperatur 15 h gerührt. Durch Zugabe von 250 ml einer 10%igen Na₂CO₃-Lösung wurde gequencht und 10 min gerührt. Die Phasen wurden getrennt und die wässrige Phase zweimal mit Methylenchlorid nachextrahiert. Die vereinigten organischen Phasen wurden mit 10%iger Na₂CO₃-Lösung und Wasser gewaschen und über Kieselgur filtriert. Das Lösungsmittel wurde im Vakuum entfernt und man erhielt 13.5 g gelblichen Feststoff.
Die HPLC-Analyse des Produktes zeigte 65,8% cis-(*2R*)-2-*tert-*Butyldiphenylsilyloxymethyl-4-(2,6-diaminopurin-9-yl)-1,3-dioxolan und 33,4% trans-(*2R*)-2-*tert*-Butyl-diphenylsilyloxymethyl-4-(2,6-diaminopurin-9-yl)-1,3-dioxolan (Isomerenverhältnis 2.0:1; HPLC Fl%). Dies entspricht einer Ausbeute an cis-Isomer von 40%.

### Beispiel 19: (Herstellung von [(2R,4R)-[4-(2,6-diamino-9H-purin-9-yl)-1,3-dioxolan-2-yl]methanol [(-)-DAPD])

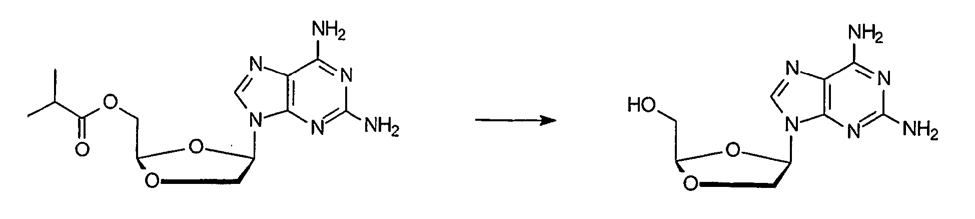

31.05 g (*2R*,*4R*)-2-Isobutyryloxymethyl-4-(2,6-diaminopurin-9-yl)-1,3-dioxolan x 2-Propanol (vgl. Beispiel 4) wurden in 310 ml NH₃-gesättigtem Methanol gelöst. Die Lösung wurde 15 h bei 25°C gerührt und das Lösungsmittel im Vakuum abdestilliert.
Der Rückstand wurde aus Ethanol / Wasser umkristallisiert. Man erhielt 17.10 g (83%) (-)-DAPD als farblose Kristalle.
¹H-NMR (360 MHz, DMSO-d₆) : δ= 3.61 (*dd,* J₁ = 6.0 Hz, J₂ = 3.2 Hz; CH₂OH) ; 4.20 (dd, J₁ = 9.5 Hz, J₂ = 5.5 Hz; 1H-C(5')) ; 4.45 (dd, J₁ = 9.5 Hz, J₂ = 1.8 Hz; 1H-C(5')) ; 5.05 (ψ*t*, J = 3.2 Hz; 1H-C(2')); 5.15 (ψ*t*, J = 6.0 Hz; CH₂OH); 5 . 83 (s; 2H-NH₂) ; 6.21 (*dd*, J₁ = 5.5 Hz, J₂ = 1.8 Hz; 1H-C(4')); 5.83 (s; 2H-NH₂) ; 7.87 (*s*; 1H-C(8)).

## Patentansprüche

1. Verfahren zur Herstellung von Verbindungen der allgemeinen Formel (1) 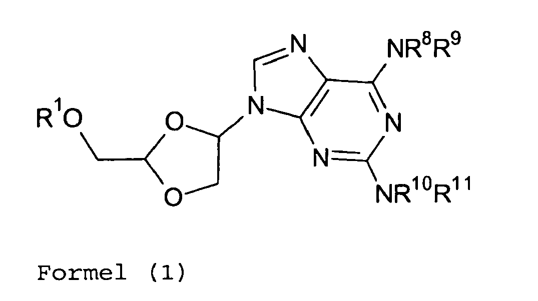 wobei
**R¹** für eine Hydroxyschutzgruppe und
**R⁸, R⁹, R¹⁰, R¹¹** unabhängig voneinander ausgewählt werden aus der Gruppe enthaltend Wasserstoff oder eine Aminoschutzgruppe
durch Umsetzung einer Verbindung der allgemeinen Formel (2) 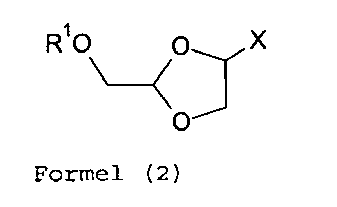 wobei
**X** für eine Abgangsgruppe steht
mit einem 2,6-Diaminopurinderivat der allgemeinen Formel (5) 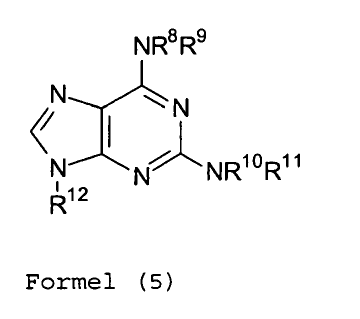 wobei
**R¹²** für einen Silylrest steht
in Gegenwart einer Lewis-Säure, **dadurch gekennzeichnet, dass**
zusätzlich eine 1,3-Dicarbonylverbindung oder ein silyliertes Derivat einer 1,3-Dicarbonylverbindung zugegen ist, wobei als 1,3-Dicarbonylverbindung ein β-Carbonylcarbonsäureester, ein 1,3-Diketon oder ein Malonsäurederivat mit 5 bis 20 C-Atomen der allgemeinen Formel (3) verwendet wird, 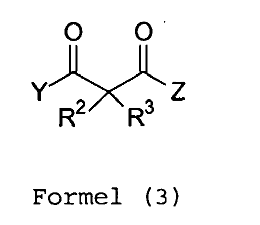 wobei
**Y** und **Z** unabhängig voneinander Wasserstoff, einen Alkylrest mit 1 bis 20 C-Atomen, einen Arylrest mit 6 bis 20 C-Atomen oder eine Alkyloxygruppe mit 1 bis 20 C-Atomen und
**R²** und **R³** unabhängig voneinander Wasserstoff, einen Acylrest einer aromatischen oder aliphatischen Carbonsäure mit 2 bis 20 C-Atomen, einen Alkylrest mit 1 bis 20 C-Atomen oder einen Arylrest mit 6 bis 20 C-Atomen
bedeuten können.

2. Verfahren nach Anspruch 1, **dadurch gekennzeichnet, dass** die Verbindungen der allgemeinen Formel (1) in der optischen Konfiguration der allgemeinen Formeln (1a), (1b), (1c) oder (1d) 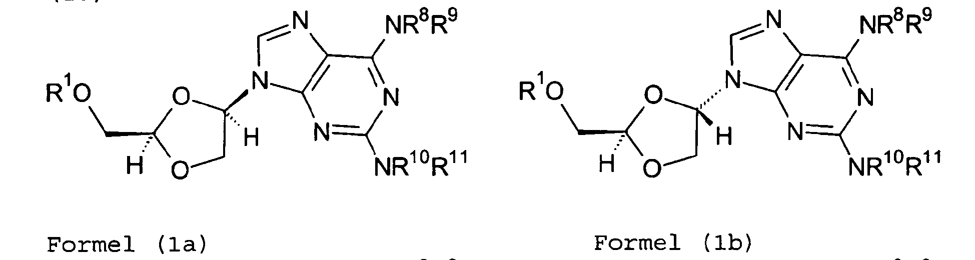 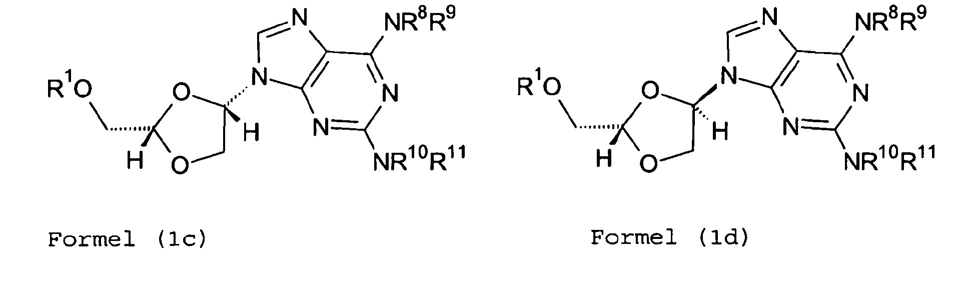 erhalten werden.

3. Verfahren nach Anspruch 1 oder 2, **dadurch gekennzeichnet, dass** R¹ ausgewählt wird aus der Gruppe enthaltend Acyl, Alkyl, Alkoxyalkyl, Arylalkyl, Arylalkoxyalkyl oder Silyl.

4. Verfahren nach einem oder mehreren der Ansprüche 1 bis 3, **dadurch gekennzeichnet, dass** X ausgewählt wird aus der Gruppe enthaltend Halogen, Acyloxy, Alkylsulfonyloxy, Arylsulfonyloxy, Alkoxy oder Aryloxy.

5. Verfahren nach einem oder mehreren der Ansprüche 1 bis 4, **dadurch gekennzeichnet, dass** als Lewis-Säure eine Verbindung ausgewählt aus der Gruppe enthaltend Trialkylsilylhalogenide oder Perfluoralkansulfonsäuretrialkylsilylester verwendet wird.

6. Verfahren nach einem oder mehreren der Ansprüche 1 bis 5, **dadurch gekennzeichnet, dass** als silyliertes Derivat einer 1,3-Dicarbonylverbindung ein Silylderivat eines β-Carbonylcarbonsäureesters, eines 1,3-Diketons oder eines Malonsäurederivates der allgemeinen Formel (4) verwendet wird, 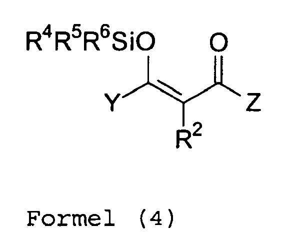 wobei
**Y, Z** und **R³** die Bedeutung nach Anspruch 1 haben und
**R⁴, R⁵** und **R⁶** unabhängig voneinander einen aliphatischen oder aromatischen Rest mit 1 bis 20 C-Atomen bedeuten können.

7. Verfahren nach einem oder mehreren der Ansprüche 1 bis 6, **dadurch gekennzeichnet, dass** die Aminoschutzgruppen ausgewählt werden aus der Gruppe enthaltend Acylreste, Acyloxycarbonylreste, Alkylreste, Arylalkylreste oder Silylreste.

8. Verfahren nach einem oder mehreren der Ansprüche 1 bis 7, **dadurch gekennzeichnet, dass** die erhaltenen Verbindungen der allgemeinen Formel (1) anschließend durch Umkristallisation gereinigt werden.

9. Verfahren nach einem oder mehreren der Ansprüche 1 bis 8, **dadurch gekennzeichnet, dass** die erhaltenen Verbindungen der allgemeinen Formel (1) durch Abspaltung der Schutzgruppen zu Verbindungen der allgemeinen Formel (6) 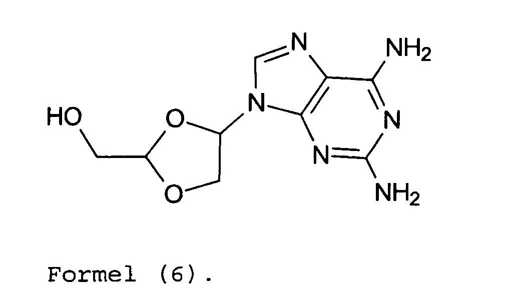 umgesetzt werden.

## Claims

1. Method for the production of compounds of the general formula (1) 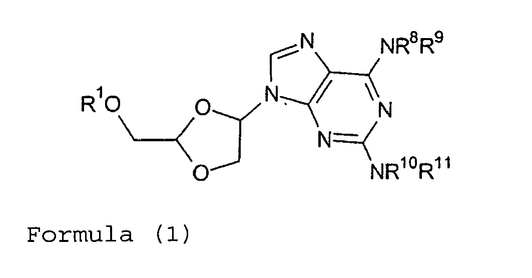 where
**R¹** is a hydroxyl protective group and
**R⁸, R⁹, R¹⁰, R¹¹** are independently of one another selected from the group comprising hydrogen or an amino protective group
by reacting a compound of the general formula (2) 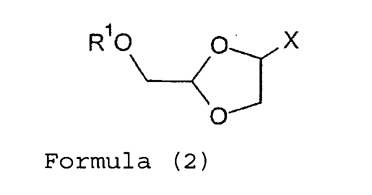 where
**X** is a leaving group,
with a 2,6-diaminopurine derivative of the general formula (5) 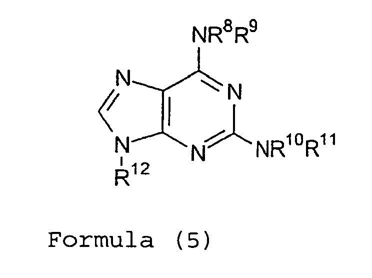 where
**R¹²** is a silyl radical,
in the presence of a Lewis acid, **characterized in that** a 1,3-dicarbonyl compound or a silylated derivative of a 1,3-dicarbonyl compound is additionally present, where the 1,3-dicarbonyl compound used is a β-carbonyl carboxylic ester, a 1,3-diketone or a malonic acid derivative having 5 to 20 C atoms of the general formula (3) 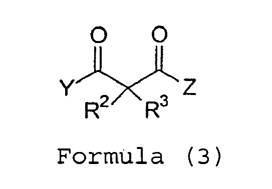 where
**Y** and **Z** may be independently of one another hydrogen, an alkyl radical having from 1 to 20 C atoms, an aryl radical having from 6 to 20 C atoms or an alkyloxy group having from 1 to 20 C atoms and
**R²** and **R³** may be independently of one another hydrogen, an acyl radical of an aromatic or aliphatic carboxylic acid having from 2 to 20 C atoms, an alkyl radical having from 1 to 20 C atoms or an aryl radical having from 6 to 20 C atoms.

2. Method according to Claim 1, **characterized in that** the compounds of the general formula (1) are obtained in the optical configuration of the general formulae (1a), (1b) , (1c) or (1d)

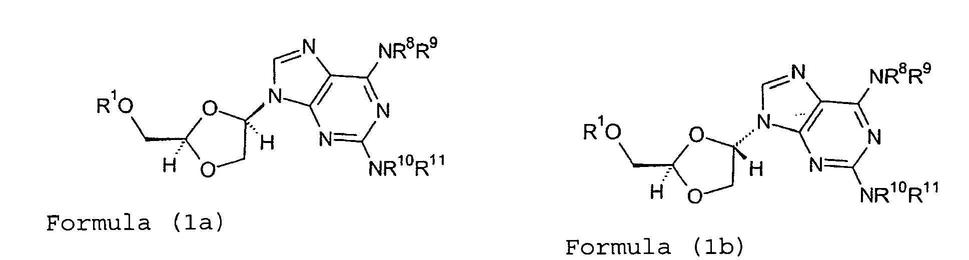

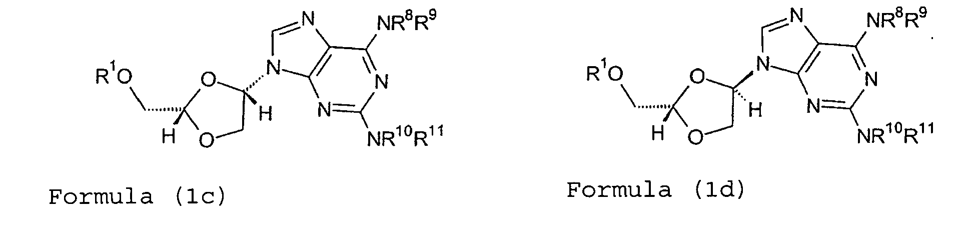

3. Method according to Claim 1 or 2, **characterized in that** R¹ is selected from the group comprising acyl, alkyl, alkoxyalkyl, arylalkyl, arylalkoxyalkyl or silyl.

4. Method according to one or more of Claims 1 to 3, **characterized in that** X is selected from the group comprising halogen, acyloxy, alkylsulfonyloxy, arylsulfonyloxy, alkoxy or aryloxy.

5. Method according to one or more of Claims 1 to 4, **characterized in that** a compound selected from the group comprising trialkylsilyl halides or trialkylsilyl perfluoroalkanesulfonates is used as Lewis acid.

6. Method according to one or more of Claims 1 to 5, **characterized in that** the silylated derivative of a 1,3-dicarbonyl compound used is a silyl derivative of a β-carbonyl carboxylic ester, of a 1,3-diketone or of a malonic acid derivative of the general formula (4) 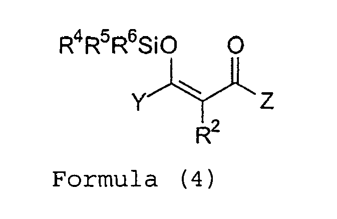 where
**Y, Z and R³** have the meaning set forth in Claim 1, and
**R⁴, R⁵ and R⁶** may be independently of one another an aliphatic or aromatic radical having from 1 to 20 C atoms.

7. Method according to one or more of Claims 1 to 6, **characterized in that** the amino protective groups are selected from the group comprising acyl radicals, acyloxycarbonyl radicals, alkyl radicals, arylalkyl radicals or silyl radicals.

8. Method according to one or more of Claims 1 to 7, **characterized in that** the resulting compounds of the general formula (1) are subsequently purified by recrystallization.

9. Method according to one or more of Claims 1 to 8, **characterized in that** the resulting compounds of the general formula (1) are reacted by eliminating the protective groups to give compounds of the general formula (6)

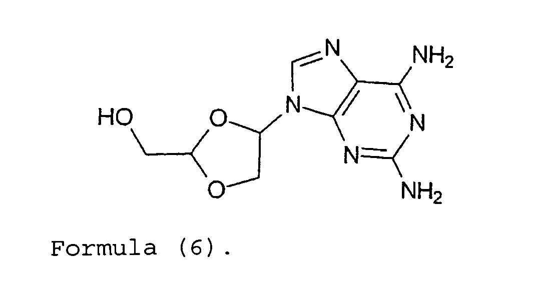

## Revendications

1. Procédé pour la préparation de composés de formule générale (1) 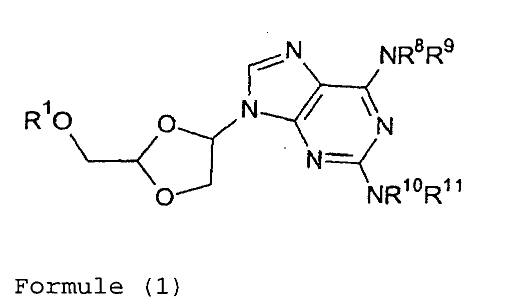 où
R¹ représente un groupement de protection de la fonction hydroxy et
R⁸, R⁹, R¹⁰, R¹¹ sont choisis, indépendamment l'un de l'autre, dans le groupe contenant hydrogène ou un groupement de protection de la fonction amino
par transformation d'un composé de formule générale (2) 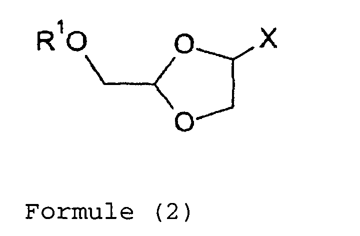 où
X représente un groupe partant avec un dérivé de 2,6-diaminopurine de formule générale (5) 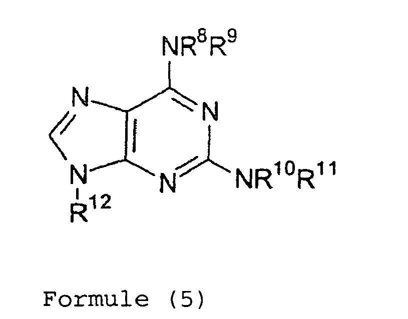 où
R¹² représente un radical silyle, en présence d'un acide de Lewis, **caractérisé en ce qu'**en outre un composé de type 1,3-dicarbonyle ou un dérivé silylé d'un composé de type 1,3-dicarbonyle est ajouté, en utilisant comme composé 1,3-dicarbonyle un ester d'acide β-carbonylcarboxylique, une 1,3-dicétone ou un dérivé d'acide malonique comprenant 5 à 20 atomes de carbone de formule générale (3), 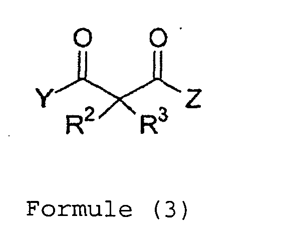 où
Y et Z peuvent signifier, indépendamment l'un de l'autre, hydrogène, un radical alkyle comprenant 1 à 20 atomes de carbone, un radical aryle comprenant 6 à 20 atomes de carbone ou un groupement alkyloxy comprenant 1 à 20 atomes de carbone et
R² et R³ peuvent signifier, indépendamment l'un de l'autre, hydrogène, un radical acyle d'un acide carboxylique aromatique ou aliphatique comprenant 2 à 20 atomes de carbone, un radical alkyle comprenant 1 à 20 atomes de carbone ou un radical aryle comprenant 6 à 20 atomes de carbone.

2. Procédé selon la revendication 1, **caractérisé en ce que** les composés de formule générale (1) sont obtenus dans la configuration optique des formules générales (1a), (1b), (1c) ou (1d)

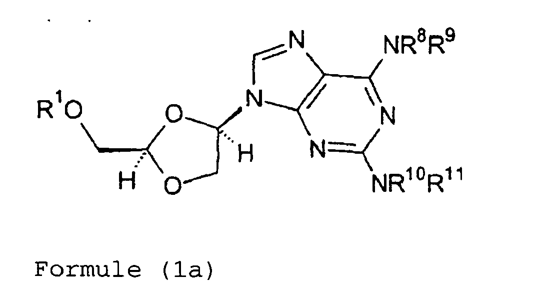

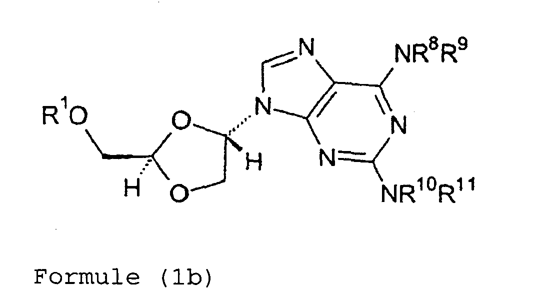

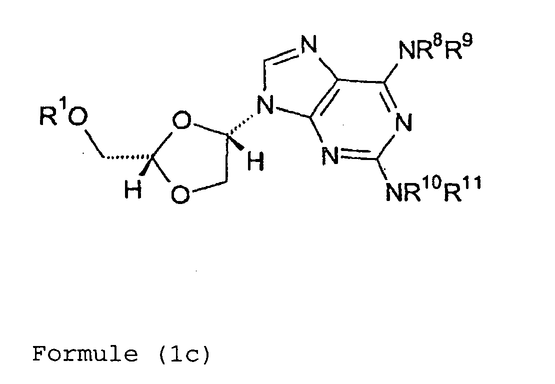

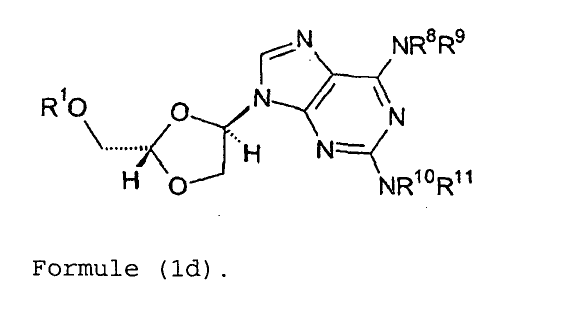

3. Procédé selon la revendication 1 ou 2, **caractérisé en ce que** R¹ est choisi dans le groupe contenant acyle, alkyle, alcoxyalkyle, arylalkyle, arylalcoxyalkyle ou silyle.

4. Procédé selon l'une ou plusieurs des revendications 1 à 3, **caractérisé en ce que** X est choisi dans le groupe contenant halogène, acyloxy, alkylsulfonyloxy, arylsulfonyloxy, alcoxy ou aryloxy.

5. Procédé selon l'une ou plusieurs des revendications 1 à 4, **caractérisé en ce qu'**on utilise comme acide de Lewis un composé choisi dans le groupe contenant les halogénures de trialkylsilyle ou les esters trialkylsilyliques de l'acide perfluoroalcanesulfonique.

6. Procédé selon l'une ou plusieurs des revendications 1 à 5, **caractérisé en ce qu'**on utilise comme dérivé silylé d'un composé de type 1,3-dicarbonyle, un dérivé silylé d'un ester d'acide β-carbonylcarboxylique, d'une 1,3-dicétone ou d'un dérivé de l'acide malonique de formule générale (4), 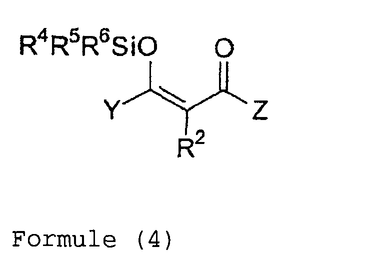 où
Y, Z et R³ ont la signification selon la revendication 1 et
R⁴, R⁵ et R⁶ peuvent signifier, indépendamment l'un de l'autre, un radical aliphatique ou aromatique comprenant 1 à 20 atomes de carbone.

7. Procédé selon l'une ou plusieurs des revendications 1 à 6, **caractérisé en ce que** les groupes de protection de la fonction amino sont choisis dans le groupe contenant les radicaux acyle, les radicaux acyloxycarbonyle, les radicaux alkyle, les radicaux arylalkyle ou les radicaux silyle.

8. Procédé selon l'une ou plusieurs des revendications 1 à 7, **caractérisé en ce que** les composés obtenus de formule générale (1) sont ensuite purifiés par recristallisation.

9. Procédé selon l'une ou plusieurs des revendications 1 à 8, **caractérisé en ce que** les composés obtenus de formule générale (1) sont transformés par dissociation des groupements de protection en composés de formule générale (6)
